(19) Europäisches Patentamt
European Patent Office
Office européen des brevets

(11) **EP 2 070 898 B1**

(12) **EUROPÄISCHE PATENTSCHRIFT**

(45) Veröffentlichungstag und Bekanntmachung des Hinweises auf die Patenterteilung:
**20.02.2013 Patentblatt 2013/08**

(51) Int Cl.:
*B01J 23/78* (2006.01)        *B01J 23/89* (2006.01)
*C07C 17/156* (2006.01)

(21) Anmeldenummer: **07122109.7**

(22) Anmeldetag: **03.12.2007**

(54) **Katalysator zur Oxichlorierung**

Catalytic converter for oxychlorination

Catalyseur d'oxychloration

(84) Benannte Vertragsstaaten:
**AT BE BG CH CY CZ DE DK EE ES FI FR GB GR HU IE IS IT LI LT LU LV MC MT NL PL PT RO SE SI SK TR**

(43) Veröffentlichungstag der Anmeldung:
**17.06.2009 Patentblatt 2009/25**

(73) Patentinhaber: **BASF SE**
**67056 Ludwigshafen (DE)**

(72) Erfinder:
• **Urtel, Heiko, Dr.**
  **67240 Bobenheim-Roxheim (DE)**
• **Junicke, Henrik, Dr.**
  **68165 Mannheim (DE)**

(56) Entgegenhaltungen:
**WO-A-2006/067188    WO-A-2006/067190**
**WO-A-2006/067191    WO-A-2006/067192**
**US-A- 5 600 043**

## Beschreibung

Die vorliegende Erfindung betrifft ein Verfahren zur Verhinderung der Aufpegelung von Wasserstoff bei Oxichlorierungsreaktionen, die in Kreisgasfahrweise durchgeführt werden.

Die Oxichlorierung von Ethylen zu 1,2-Dichlorethan (EDC) ist ein allgemein bekanntes Verfahren, bei dem Ethylen mit Chlorwasserstoff und Sauerstoff oder mit einem sauerstoffhaltigen Gas (z.B. Luft) in der Gasphase und üblicherweise in Gegenwart eines Katalysators umgesetzt wird. Dabei wird häufig Chlorwasserstoff aus der Pyrolysereaktion von 1,2-Dichlorethan (EDC) eingesetzt, der generell Verunreinigungen (insbesondere Acetylen) enthält. Diese führen bei der Oxichlorierungsreaktion zu Nebenprodukten (insbesondere polychlorierte Verbindungen), wodurch die Abtrennung des Hauptprodukts EDC aus der Oxichlorierungsreaktion erschwert wird. Daher wird der Chlorwasserstoff vor der Rückführung in die Oxichlorierungsreaktion einem Hydrierungsschritt unterzogen, bei dem die Verunreinigungen zersetzt werden. Dazu wird der Chlorwasserstoff, mit einem bis zu fünffachen Überschuss an Wasserstoff - bezogen auf die zu hydrierenden Verunreinigungen (insbesondere Acetylen) - in Gegenwart eines Hydrierungskatalysators zur Reaktion gebracht. Der für die Oxichlorierung eingesetzte Chlorwasserstoff enthält daher auch Wasserstoff.

In der DE 1 618 701 ist ein Verfahren zur Oxichlorierung beschrieben, bei dem das Reaktionsgas im Kreis geführt wird.

In der als "Mitsui-Toatsu-Verfahren" bekannten Durchführungsweise der Oxichlorierung wird Ethylen in Gegenwart von Sauerstoff und Chlorwasserstoff an einem Wirbelbettkatalysator aus Aluminiumoxid und Kupfer umgesetzt, wobei die molaren Verhältnisse des Reaktionsgases Ethylen 1,5 bis 2,00 : Chlorwasserstoff 2,00 : Sauerstoff 0,5 bis 1,00 betragen. Das aus dem Wirbelschichtreaktor austretende Gas wird dabei mittels eines Kühlers oder einer anderen geeigneten Vorrichtung in Produkte und eine gasförmige Mischung, die nicht umgesetzte Edukte und gasförmige Nebenprodukte wie zum Beispiel Kohlendioxid und Kohlenmonoxid enthält, aufgetrennt. Aus dem Gasgemisch wird durch Waschen mit einer basischen Lösung Kohlendioxid und eventuell restlicher Chlorwasserstoff entfernt. Das so erhaltene Gasgemisch, welches hauptsächlich Ethylen, Sauerstoff und Kohlenmonoxid enthält, wird im Kreislauf zum Wirbelbettreaktor zurückgeführt. Vor dem erneuten Eintritt in den Wirbelbettreaktor werden dem Kreisgas Ethylen, Sauerstoff und Chlorwasserstoff zugesetzt. Bei diesem Verfahren werden hohe Chlorwasserstoffumsätze erreicht, die erzielten EDC-Selektivitäten sind im Allgemeinen jedoch nicht zufrieden stellend.

Es ist beschrieben, dass durch Dotierung des Kupfer-enthaltenden Katalysators mit Magnesium und/oder Kalium die EDC-Selektivität der Oxichlorierung verbessert werden kann. Beispiele für solche Katalysatoren sind etwa in EP 1 464 395 und EP 1 666 145 für den Einsatz in *"Single Pass"* Reaktoren beschrieben, wobei die dort verwendeten Katalysatoren neben der Dotierung mit Magnesium und Kalium noch eine spezielle Konzentrationsverteilung des Katalysatormetalls über den Katalysatorträgerpartikel aufweisen. Unter *"Single Pass"* Verfahren werden in der vorliegenden Erfindung Verfahren verstanden, bei denen das Reaktionsgas den Reaktor nur einmal durchläuft und nicht in einem Kreisprozess geführt wird.

Für ein wirtschaftliches Verfahren ist es jedoch wünschenswert, dass dieses in einem Kreisprozess durchgeführt wird, nicht umgesetzte Edukte also zum Reaktor zurückgeführt werden.

Die im Stand der Technik beschriebenen Katalysatoren, die eine hohe EDC-Selektivität aufweisen, sind generell nicht für Oxichlorierungsreaktionen in Kreisgasfahrweise geeignet. Aufgrund der hohen Selektivität wird eine Aufpegelung von Wasserstoff beobachtet, da dieser trotz seiner prinzipiell hohen Reaktivität nicht zu Folgeprodukten (im Allgemeinen Wasser) reagiert und sich durch die Kreisgasfahrweise anreichert. Durch den hohen Wasserstoffgehalt resultieren unsichere Betriebszustände, die Gefahr etwa von Explosionen wächst.

Bei den bisher gebräuchlichen Oxichlorierungsverfahren in Kreisgasfahrweise werden daher Katalysatoren eingesetzt, die Kupfer in Konzentrationen von 10 bis 12 Gew.-% enthalten, eine hohe Aktivität besitzen und somit einen hohen Chlorwasserstoffumsatz bewirken. Aufgrund der hohen Aktivität wird neben der Reaktion von Ethylen zu EDC auch die Umsetzung (Abbau) von Wasserstoff zu Wasser in ausreichendem Maß katalysiert. Die hohe Aktivität des Katalysators geht jedoch im Allgemeinen einher mit nicht zufriedenstellenden EDC-Selektivitäten; es wird etwa auch die Bildung von unerwünschten Nebenprodukten, wie zum Beispiel Kohlenmonoxid und Kohlendioxid katalysiert.

Es bestand somit die Aufgabe, ein Verfahren zur Oxichlorierung in Kreisgasfahrweise bereitzustellen, bei dem die Aufpegelung von Wasserstoff im Reaktionsgas verhindert oder zumindest stark reduziert wird. Unsichere Betriebszustände sollen vermieden werden. Die EDC-Selektivität soll bei gleichzeitig hohem Chlorwasserstoffumsatz im Vergleich zu aus dem Stand der Technik bekannten, ebenfalls im Kreisprozess betriebenen Oxichlorierungsverfahren verbessert werden. Diese soll vergleichbar sein mit Selektivitäten, die von Katalysatoren aus dem Stand der Technik bekannt sind. Das Verfahren soll möglichst einfach und wirtschaftlich sein, am besten durch Einsatz eines Katalysators, der vorzugsweise einfach herstellbar und kostengünstig ist.

Gelöst wird die Aufgabe durch ein Verfahren zur Oxichlorierung von Ethylen zu 1,2-Dichlorethan in Kreisgasfahrweise mit verminderter Wasserstoffaufpegelung mit folgenden Schritten:

(A) Einleiten von Ethylen, Sauerstoff und Chlorwasserstoff als Edukte in einen Oxichlorierungsreaktor, wobei der Chlorwasserstoff Wasserstoff enthält,

(B) Umsetzen der Edukte an einem Katalysator enthaltend

■ 3,5 bis 12,5 Gew.-% Kupfer als Kupfersalz
■ 0,1 bis 1,0 Gew.-% Magnesium als Magnesiumsalz
■ 0,1 bis 2,0 Gew.-% Kalium als Kaliumsalz auf einer Trägersubstanz, zu 1,2-Dichlorethan und Wasser unter zumindest teilweiser Umsetzung des vorhandenen Wasserstoffs zu Wasser,

(C) Aufarbeitung des Reaktoraustrags und Rückführung des Kreisgases zum Oxichlorierungsreaktor.

Im Rahmen der vorliegenden Erfindung sind alle Gewichtsprozentangaben auf das Gesamtgewicht des Katalysators einschließlich der Trägersubstanz bezogen.

Unter Reaktionsgas wird in der vorliegenden Erfindung immer die Summe von Frischgas und Kreisgas verstanden; unter Kreisgas ist jenes Gasgemisch zu verstehen, das nach dem Reaktordurchgang und gegebenenfalls nach Aufarbeitungsschritten erhalten wird und zum Reaktor zurückgeführt wird.

Der Chlorwasserstoffumsatz ist ein Maß, für die Aktivität des im Verfahren eingesetzten Katalysators und ist definiert als:

Chlorwasserstoffumsatz [%] = {umgesetzter Chlorwasserstoff / frisch zugeführter Chlorwasserstoff} • 100

Die EDC-Selektivität ist definiert als:

EDC-Selektivität [%] = {gebildetes EDC / umgesetztes Ethylen} • 100

Um die Aufpegelung des Wasserstoffs im Reaktionsgas zu verhindern, muss beim einfachen Reaktordurchgang eine Wasserstoff-Menge abgebaut werden, die mindestens so groß ist wie die jeweils frisch zugeführte Wasserstoffmenge.

Im Rahmen der vorliegenden Erfindung wird unter verminderter Wasserstoffaufpegelung verstanden, dass die Wasserstoffaufpegelung entweder vollständig verhindert wird, d.h. dass beim einfachen Reaktordurchgang Wasserstoff vollständig abgebaut wird und im Kreisgas kein Wasserstoff vorhanden ist, oder dass der Wasserstoff beim einfachen Reaktordurchgang in einem Maß abgebaut wird, dass sich der Wasserstoffgehalt im Kreisgas bei Werten einpegelt, die sichere Betriebszustände ermöglichen und zu einem Wasserstoffgehalt im Reaktionsgas von maximal 2,0%, bevorzugt zu Werten von maximal 1,8 und insbesondere bevorzugt zu Werten von 1,5 % führen

Es wurde überraschenderweise gefunden, dass durch den Einsatz von Katalysatoren, die Kupfer, Magnesium und Kalium in Mengenverhältnissen innerhalb des erfindungsgemäßen Bereichs enthalten, hohe Produktselektivitäten erreicht werden und gleichzeitig die Reaktion von Wasserstoff zu Wasser in ausreichendem Maß katalysiert wird. Bei Kreisgasfahrweise wird so eine Aufpegelung von Wasserstoff verhindert, so dass unsichere Betriebszustände vermieden werden. Durch den Einsatz dieser Katalysatoren wird die Bildung von unerwünschten Nebenprodukten unterdrückt, und es werden gute EDC-Selektivitäten bei hohem Chlorwasserstoffumsatz erreicht.

Das erfindungsgemäße Verfahren verbindet somit die Verhinderung der Aufpegelung von Wasserstoff im Reaktionsgas mit guter EDC-Selektivität bei gleichzeitig hohem Chlorwasserstoffumsatz. Darüber hinaus erhöht das erfindungsgemäße Verfahren die Prozesssicherheit.

Der im erfindungsgemäßen Verfahren eingesetzte Katalysator enthält Metallsalze in solchen Verhältnisanteilen auf einer Trägersubstanz, dass er 3,5 bis 12,5 Gew.-% Kupfer als Kupfersalz, 0,1 bis 1,0 Gew.-% Magnesium als Magnesiumsalz und 0,1 bis 2,0 Gew.-% Kalium als Kaliumsalz umfasst, wobei alle Gewichtsprozente auf das Gesamtgewicht des Katalysators einschließlich der Trägersubstanz bezogen sind.

In einer bevorzugten Ausführungsform enthält der im erfindungsgemäßen Verfahren eingesetzte Katalysator Metallsalze in solchen Verhältnissen auf einer Trägersubstanz, dass er 7,5 bis 9,0 Gew.-% Kupfer als Kupfersalz, 0,3 bis 0,7 Gew.-% Magnesium als Magnesiumsalz und 0,6 bis 0,9 Gew.-% Kalium als Kaliumsalz umfasst, wobei alle Gewichtsprozente auf das Gesamtgewicht des Katalysators einschließlich der Trägersubstanz bezogen sind.

Gegebenenfalls enthält der im erfindungsgemäßen Verfahren eingesetzte Katalysator weitere Metalle in Form von Metallsalzen oder in elementarer Form, wie zum Beispiel zusätzliche Alkalimetalle, zusätzliche Erdalkalimetalle, Metalle der seltenen Erden, Übergangsmetalle und Edelmetalle wie Gold, Ruthenium, Platin und Palladium.

Die spezifische Oberfläche (BET) des im erfindungsgemäßen Verfahren eingesetzten Katalysators liegt vorzugsweise im Bereich von 60 bis 200 $m^2$/g, mehr bevorzugt bei 80 bis 145 $m^2$/g, insbesondere im Bereich von 90 bis 130 $m^2$/g (die Bestimmung der BET Oberfläche erfolgt nach DIN 66131). Das Porenvolumen des Wirbelschichtkatalysators liegt im Bereich von 0,2 bis 0,5 $cm^3$/g (die Bestimmung des Porenvolumens erfolgt nach DIN 66133). Das Stampfgewicht des erfindungsgemäßen Katalysators liegt im Bereich von 900 bis 1200, vorzugsweise im Bereich von 950 bis 1150. Besonders bevorzugt sind Katalysatoren mit einem Stampfgewicht größer 1000 g/l. Die mittlere Partikelgröße (D50-Wert) des Katalysators, liegt bei Werten von 45 bis 75 μm.

Die Einstellung der Metallkonzentrationen, bezogen auf das Gesamtgewicht des Katalysators einschließlich Trägersubstanz, erfolgt nach bekannten, dem Fachmann geläufigen Methoden, durch Imprägnierung oder Mischfällung. Vorzugsweise erfolgt die Einstellung der Metallkonzentration durch Imprägnierung.

Zur Imprägnierung werden die erforderlichen Mengen der Metallsalzverbindungen, vorzugsweise in Form ihrer Chloride, Nitrate, Acetate, Hydroxide oder Carbonate in Wasser gelöst. Besonders bevorzugt ist der Einsatz von Metallchloridverbindungen. Die wässrige Lösung wird auf die Trägersubstanz aufgebracht. Die Trägersubstanz wird gegebenenfalls abfiltriert und getrocknet. Vorzugsweise wird die Wassermenge so gewählt, dass sie ca. 70 bis 90 % der Wasseraufnahme der Trägersubstanz entspricht. In diesem Fall ist eine Filtration nicht notwendig.

Die Trocknung erfolgt bei Raumtemperatur oder bei Temperaturen im Bereich von 100 bis 200 °C in Gegenwart von Luft oder Schutzgas. Bevorzugt ist die Trocknung in Gegenwart von Stickstoff bei Temperaturen im Bereich von 110 bis 180 °C.

Bei der Durchführung des erfindungsgemäßen Verfahrens zur Herstellung von 1,2-Dichlorethan können die bereits bekannten Techniken und Reaktionsbedingungen, die nach dem Stand der Technik allgemein eingeführt sind, verwendet werden. Dazu werden Ethylen, Chlorwasserstoff und Sauerstoff mit dem erfindungsgemäßen Katalysator in der Gasphase in Kontakt gebracht.

Für das erfindungsgemäße Verfahren eignen sich alle Oxichlorierungsreaktoren, bei denen das Reaktionsgas im Kreis geführt wird. Geeignete Reaktoren sind Rohrreaktoren, Festbettreaktoren und Wirbelschichtreaktoren. Bevorzugte sind Wirbelschichtreaktoren.

Nach dem erfindungsgemäßen Verfahren liegt die Zusammensetzung des Reaktionsgases im Bereich der folgenden molaren Verhältnisse: Ethylen 1,0 bis 2,0 : Chlorwasserstoff 2,0 : Sauerstoff 0,5 bis 1,0.

In einer bevorzugten Ausführungsform des erfindungsgemäßen Verfahren liegt die Zusammensetzung des Reaktionsgases im Bereich der folgenden molaren Verhältnisse: Ethylen 1,55 bis 2,0 : Chlorwasserstoff 2,0 : Sauerstoff 0,5 bis 0,75.

Als sauerstoffhaltiges Gas eignen sich Luft oder Sauerstoff, bevorzugt ist Sauerstoff. Als Chlorwasserstoff kann prinzipiell Chlorwasserstoff aus den üblichen Quellen eingesetzt werden. Aus praktischen Gründen ist Chlorwasserstoff bevorzugt, der aus der EDC-Pyrolyse gewonnen wird und Wasserstoffkonzentrationen von 0 bis 2 Vol.-% enthält.

Die Temperatur im Reaktor liegt allgemein bei Werten von 80 bis 300°C, bevorzugt bei Werten von 200 bis 280°C, insbesondere bei Werten von 210 bis 260 °C.

Der Druck im Reaktor liegt bei Werten von 1 bis 20 bar, bevorzugt bei Werten von 1 bis 8 bar, insbesondere bei Werten von 1 bis 5 bar.

Geeignete Trägersubstanzen für den im erfindungsgemäßen Verfahren eingesetzten Katalysator sind Aluminiumoxide, wie alpha-Aluminiumoxid, beta-Aluminiumoxid und gamma-Aluminiumoxid, aktivierte Aluminiumoxide, Siliciumoxide wie Silikate, Silikagele, Kieselsäure und Kieselgur, Graphit, Metalloxide, Zirkonoxide, Zeolithe, Wasserglas, Bimsstein, Tone, Korunde und Mischungen der vorstehend genannten Substanzen. Bevorzugt ist der Einsatz von gamma-Aluminiumoxid.

Die spezifische Oberfläche der Trägersubstanz vor der Metallsalz-Ablagerung liegt vorzugsweise im Bereich von 20 bis 400 $m^2$/g, mehr bevorzugt bei Werten von 75 bis 200 $m^2$/g (die Bestimmung der BET-Oberfläche erfolgt nach DIN 66131). Gebräuchliche Trägersubstanzen verfügen vorzugsweise über Porenvolumina im Bereich von 0,15 bis 0,75 $cm^3$/g (die Bestimmung des Porenvolumens wird nach DIN 66133 durchgeführt). Das Stampfgewicht der Trägersubstanz liegt im Bereich von 600 bis 950, vorzugsweise im Bereich von 700 bis 850 (Das Stampfgewicht ist ein Maß für die Dichte partikulärer Feststoffe. Die Bestimmung des Stampfgewichts erfolgt auf einem STAV 2003 Stampfvolumeter der Firma JEL. Das Einfüllen des Probenmaterials wird während der ersten 350 Rüttelcyclen vorgenommen. Das Stampfvolumen wird nach weiteren 350 Cyclen bestimmt). Die mittlere Partikelgröße (D50-Wert) der Trägersubstanz liegt bei Werten von 45 bis 75 μm (die Bestimmung der Partikelgrößen erfolgt mit einem Analysegerät Mastersizer S von der Firma Malvern; Messparameter für das Gerät, Gasgeschwindigkeit 80 m/s, Streumodell 3 $$ A (Fraunhofer) Brennweite 300 mm, Strahlenlänge 10 mm, Dispergierdruck 0,5 bar; Iso 13320).

In einer besonderen Ausführungsform des erfindungsgemäßen Verfahrens wird die Oxichlorierung in einem Wirbelschichtreaktor an einem Wirbelschichtkatalysator durchgeführt, wobei die molaren Verhältnisse des Reaktionsgases Ethlyen 1,55 bis 2,00 :

Chlorwasserstoff 2,00 : Sauerstoff 0,5 bis 0,75 betragen. Das aus dem Wirbelschichtreaktor austretende Gas wird dabei mittels eines Kühlers oder einer anderen geeigneten Vorrichtung in Produkte und eine gasförmige Mischung, die nicht umgesetzte Edukte und gasförmige Nebenprodukte wie zum Beispiel Kohlendioxid und Kohlenmonoxid enthält, aufgetrennt. Aus dem Gasgemisch wird durch Waschen mit einer basischen Lösung Kohlendioxid und eventuell restlicher Chlorwasserstoff entfernt. Das so erhaltene Gasgemisch, welches hauptsächlich Ethylen, Sauerstoff und Kohlenmonoxid enthält wird im Kreislauf zum Wirbelbettreaktor zurückgeführt. Vor dem erneuten Eintritt in den Wirbelbettreaktor werden dem Kreisgas Ethylen, Sauerstoff und Chlorwasserstoff in solchen Mengen zugesetzt, dass das Reaktionsgas die oben beschriebenen molaren Verhältnisse aufweist.

Die Erfindung wird durch die nachfolgenden Beispiele näher erläutert, ohne darauf beschränkt zu sein.

**Beispiele:**

Die im erfindungsgemäßen Verfahren eingesetzten Katalysatoren wurden durch Imprägnierung der Trägersubstanz mit einer wässrigen Lösung wie in der Folge beschrieben erhalten:

Die zur Einstellung der gewünschten Metallverhältnisse notwendigen Mengen der entsprechenden Metallsalzchloride wurden in einer kleinen Menge Wasser gelöst. Weiteres Wasser wurde bis auf ein Gesamtvolumen von ca. 90 % der maximalen Wassernahme des eingesetzten Trägermaterials aufgefüllt. Diese Lösung wurde in einer Tränktrommel auf einen gamma-Aluminiumoxidträger mit einer BET-Oberfläche von 170 $m^2$/g aufgesprüht. Die mittleren Partikelgrößen der Träger (d50-Wert) lagen zwischen 45 und 75 $\mu$m. Nach beendeter Zugabe wurde 16 h in Gegenwart von Stickstoff bei 110-180 °C getrocknet.

Zur Durchführung von Vergleichsversuchen wurden die erfindungsgemäßen Katalysatoren 1 bis 3 und die Vergleichskatalysatoren 4 bis 8 in oben beschriebener Weise hergestellt. Die Zusammensetzungen der Katalysatoren sind in Tabelle 1 zusammengefasst.

**Tabelle 1**

| | Beispiele | | | Vergleichsbeispiel | | | | |
|---|---|---|---|---|---|---|---|---|
| Katalysator | 1 | 2 | 3 | 4 | 5 | 6 | 7 | 8 |
| Cu [Gew.-%] | 6,0 | 8,1 | 8,2 | 8,0 | 10,5 | 5,8 | 9,9 | 12 |
| Mg [Gew.-%] | 0,1 | 0,9 | 0,49 | 1,5 | 1,75 | 1,4 | - | - |
| K [Gew.-%] | 0,8 | 0,7 | 0,83 | 0,8 | 0,85 | 0,81 | 0,93 | - |
| Au [Gew.-%] | 0,0009 | 0,0009 | - | - | - | 0,010 | - | - |
| BET [$m^2$/g] | 112 | 110 | 115 | 112 | 92 | 105 | 141 | 260 |

Bei den Reaktoren handelt es sich um ölbeheizte Glasreaktoren mit 3 bzw. 4 cm Reaktor-Innendurchmesser.

Die Reaktionstemperatur wurde über den Hot-Spot des Reaktors mittels eines Thermofühlers gemessen und gesteuert. Alle Versuche wurden mit jeweils fixen Einstellungen der Gasflüsse bei den angefahrenen Temperaturen durchgeführt.

Zur Bestimmung des Wasserstoffumsatzes wurde ein Reaktor mit 4 cm Innendurchmesser, der mit 240 g Katalysator beschickt war, verwendet. Die Reaktion wurde bei einem Druck von 2,4 bar bei einer Chlorwasserstoff-Belastung von 425 NI/kg Kat/h durchgeführt. Als Reaktionsgas wurde eine Mischung folgender Zusammensetzung verwendet: Ethylen 23,2 Vol.-%, Wasserstoff 0,95 Vol.-%, Sauerstoff 9,1 Vol.-% :

Chlorwasserstoff 28,1 Vol.-%, Differenz Stickstoff. Dies entspricht einer Stöchiometrie von Ethylen : Chlorwasserstoff : Sauerstoff von 1,65 : 2: 0,65 ($\pm$ 0,02). Die Ergebnisse sind in Tabelle 2 zusammengefasst.

**Tabelle 2**

| Katalysator | | 3 | 6 | 7 | 8 |
|---|---|---|---|---|---|
| Cu | [Gew.-%] | 8,2 | 5,8 | 9,9 | 12,0 |
| Mg | [Gew.-%] | 0,49 | 1,40 | - | - |
| K | [Gew.-%] | 0,83 | 0,81 | 0,93 | - |

(fortgesetzt)

| Katalysator | | 3 | 6 | 7 | 8 |
|---|---|---|---|---|---|
| Au | [Gew.-%] | - | 0,010 | - | - |
| BET | [m$^2$/g] | 115 | 105 | 141 | 260 |
| Umsatz HCl 220 °C | [%] | 98,7 | 70,2 | 99,7 | 97,4 |
| HCl-Konz. im Abgas | [Vol.-%] | 0,5 | 11,7 | 0,1 | 1,1 |
| H$_2$-Umsatz 220 °C | [%] | 9,0 | 2,2 | 20,0 | 14,5 |
| Umsatz HCl 230 °C | [%] | 99,2 | 73,7 | 99,4 | 95,5 |
| HCl-Konz. im Abgas | [Vol.-%] | 0,3 | 10,4 | 0,2 | 1,8 |
| H$_2$-Umsatz 230 °C | [%] | 9,9 | 1,2 | 27,7 | 13,8 |
| Umsatz HCl 245 °C | [%] | 99,5 | 87,9 | 98,6 | 87,0 |
| HCl-Konz. im Abgas | [Vol.-%] | 0,2 | 5,0 | 0,5 | 5,0 |
| H$_2$-Umsatz 245 °C | [%] | 21,7 | 8,3 | 32,5 | 14,8 |

Die Versuche zeigen, dass der erfindungsgemäße Katalysator 3 im Vergleich zu Katalysator 6 deutlich höhere Wasserstoffabbauraten erreicht. Die Wasserstoffabbauraten von Katalysator 3 sind zwar niedriger, als die der Katalysatoren 7 und 8, allerdings ausreichend hoch, um die Aufpegelung von Wasserstoff im Kreisgas zu verhindern.

Zur Bestimmung der EDC-Selektivität wurde ein Reaktor mit 4 cm Innendurchmesser, der mit 240 g Katalysator beschickt war, verwendet. Die Reaktion wurde bei einem Druck von 2,4 bar bei einer Chlorwasserstoff-Belastung von 500 Nl/kg Kat/h durchgeführt. Als Reaktionsgas wurde eine Mischung folgender Zusammensetzung verwendet: Ethylen 24,6 Vol.-%, Sauerstoff 9,7 Vol.-% : Chlorwasserstoff 29,9 Vol.-%, Differenz Stickstoff. Dies entspricht einer Stöchiometrie von Ethylen : Chlorwasserstoff : Sauerstoff von 1,65 : 2: 0,65 ($\pm$ 0,02). Die Ergebnisse sind in Tabelle 3 zusammengefasst.

**Tabelle 3**

| Katalysator | | 3 | 6 | 7 | 8 |
|---|---|---|---|---|---|
| Cu | [Gew.-%] | 8,2 | 5,8 | 9,90 | 12,00 |
| Mg | [Gew.-%] | 0,49 | 1,40 | - | - |
| K | [Gew.-%] | 0,83 | 0,81 | 0,93 | - |
| Au | [Gew.-%] | - | 0,010 | - | - |
| BET | [m$^2$/g] | 115 | 105 | 141 | 260 |
| *215 °C* | | | | | |
| Umsatz HCl | [%] | 99,0 | 66,4 | | 99,7 |
| HCl-Konz. im Abgas | [Vol.-%] | 0,3 | 10,5 | | 0,1 |
| EDC-Selektivität | [%] | 99,58 | 99,54 | | 94,58 |
| Ausbeute (CO+CO$_2$) | [%] | 0,1 | <0,1 | | 2,3 |
| *230 °C* | | | | | |
| Umsatz HCl | [%] | 97,0 | 78,6 | | 99,0 |
| HCl-Konz. im Abgas | [Vol.-%] | 0,9 | 6,4 | | 0,3 |
| EDC-Selektivität | [%] | 99,49 | 99,56 | | 92,5 |
| S (CO+CO$_2$) | [%] | 0,1 | <0,1 | | 3,8 |

(fortgesetzt)

| 235 °C | | | | | |
|---|---|---|---|---|---|
| Umsatz HCl | [%] | 97,6 | 80,7 | 98,2 | 98,3 |
| HCl-Konz. im Abgas | [Vol.-%] | 0,2 | 5,8 | 0,6 | 0,5 |
| EDC-Selektivität | [%] | 98,3 | 99,12 | 95,1 | 87,1 |
| S (CO+CO$_2$) | [%] | 0,39 | <0,1 | 1,0 | 3,84 |
| 240 °C | | | | | |
| Umsatz HCl | [%] | 96,3 | 83,6 | | 96,7 |
| HCl-Konz. im Abgas | [Vol.-%] | 1,1 | 4,9 | | 1,0 |
| EDC-Selektivität | [%] | 99,14 | 99,56 | | 89,7 |
| S (CO+CO$_2$) | [%] | 0,31 | <0,1 | | 4,5 |
| 255 °C | | | | | |
| Umsatz HCl | [%] | 95,0 | 87,6 | | 89,0 |
| HCl-Konz. im Abgas | [Vol.-%] | 1,5 | 3,7 | | 3,3 |
| EDC-Selektivität | [%] | 98,28 | 98,48 | | 80,7 |
| S (CO+CO$_2$) | [%] | 0,75 | 0,3 | | 4,6 |

Die Versuche zeigen, dass der erfindungsgemäße Katalysator 3 über einen weiten Temperaturbereich im Vergleich zu den Katalysatoren 7 und 8 deutlich höhere EDC-Selektivitäten bei hohem Chlorwasserstoffumsatz erreicht und Wasserstoff in ausreichendem Maß abbaut.

**Patentansprüche**

1. Verfahren zur Oxichlorierung von Ethylen zu 1,2-Dichlorethan in Kreisgasfahrweise mit verminderter Wasserstoffaufpegelung mit folgenden Schritten:

(A) Einleiten von Ethylen, Sauerstoff und Chlorwasserstoff als Edukte in einen Oxichlorierungsreaktor, wobei der Chlorwasserstoff Wasserstoff enthält,
(B) Umsetzen der Edukte an einem Katalysator enthaltend

• 3,5 bis 12,5 Gew.-% Kupfer als Kupfersalz
• 0,1 bis 1,0 Gew.-% Magnesium als Magnesiumsalz
• 0,1 bis 2,0 Gew.-% Kalium als Kaliumsalz
auf einer Trägersubstanz,

zu 1,2-Dichlorethan und Wasser unter zumindest teilweiser Umsetzung des vorhandenen Wasserstoffs zu Wasser,
(C) Aufarbeitung des Reaktoraustrags und Rückführung des Kreisgases zum Oxichlorierungsreaktor.

2. Verfahren gemäß Anspruch 1, **dadurch gekennzeichnet, dass** der Katalysator 7,5 bis 9,0 Gew.-% Kupfer als Kupfersalz, 0,3 bis 0,7 Gew.-% Magnesium als Magnesiumsalz und 0,6 bis 0,9 Gew.-% Kalium als Kaliumsalz auf einer Trägersubstanz enthält.

3. Verfahren gemäß einem der Ansprüche 1 bis 2, **dadurch gekennzeichnet, dass** die Trägersubstanz des Katalysators ausgewählt ist aus der Gruppe Aluminiumoxide, alpha-Aluminiumoxid, beta-Aluminiumoxid, gamma-Aluminiumoxid, aktivierte Aluminiumoxide, Siliciumoxide wie Silikate, Silikagele, Kieselsäure und Kieselgur, Graphit, Metalloxide, Zirkonoxide, Zeolithe, Wasserglas, Bimsstein, Tone, Korunde oder Mischungen der vorstehend genannten Substanzen.

**4.** Verfahren gemäß einem der Ansprüche 1 bis 3, **dadurch gekennzeichnet, dass** der Katalysator als Trägersubstanz gamma-Aluminiumoxid enthält.

**5.** Verfahren gemäß einem der Ansprüche 1 bis 4, **dadurch gekennzeichnet, dass** der Katalysator eine BET Oberfläche im Bereich von 60 bis 200 m$^2$/g aufweist.

**6.** Verfahren gemäße einem der Ansprüche 1 bis 5, **dadurch gekennzeichnet, dass** der Katalysator eine BET Oberfläche im Bereich von 80 bis 145 m$^2$/g aufweist.

**7.** Verfahren gemäß einem der Ansprüche 1 bis 6, **dadurch gekennzeichnet, dass** der Katalysator eine BET Oberfläche im Bereich von 100 bis 130 m$^2$/g aufweist.

**8.** Verfahren gemäß einem der Ansprüche 1 bis 7, **dadurch gekennzeichnet, dass** der Katalysator ein Stampfgewicht größer 1000 g/l aufweist.

**9.** Verfahren gemäß einem der Ansprüche 1 bis 8, **dadurch gekennzeichnet, dass** der Katalysator eine mittlere Partikelgröße (D$_{50}$-Wert) im Bereich von 45 bis 75 $\mu$m aufweist.

**10.** Verfahren gemäß einem der Ansprüche 1 bis 9, **dadurch gekennzeichnet, dass** die Edukte in den molaren Verhältnissen Ethylen 1,00 bis 2,0 : Chlorwasserstoff 2,0 : Sauerstoff 0,5 bis 1,0 in den Reaktor eingeleitet werden.

**11.** Verfahren gemäß einem der Ansprüche 1 bis 10, **dadurch gekennzeichnet, dass** die Edukte in den molaren Verhältnissen Ethylen 1,55 bis 2,0 : Chlorwasserstoff 2,0 : Sauerstoff 0,5 bis 0,75 in den Reaktor eingeleitet werden.

**12.** Verfahren gemäß einem der Ansprüche 1 bis 11, **dadurch gekennzeichnet, dass** der Oxichlorierungsreaktor ein Wirbelschichtreaktor ist.

**13.** Verwendung eines Katalysators enthaltend

■ 3,5 bis 12,5 Gew.-% Kupfer als Kupfersalz,
■ 0,1 bis 1 Gew.-% Magnesium als Magnesiumsalz und
■ 0,1 bis 2 Gew.-% Kalium als Kaliumsalz
auf einer Trägersubstanz

zur Verhinderung der Aufpegelung von Wasserstoff in einem Verfahren zur Oxichlorierung von Ethylen zu 1,2-Dichlorethan in Kreisgasfahrweise wie in einem der Ansprüche 1 bis 12 beschrieben.

**Claims**

**1.** A process for the oxychlorination of ethylene to form 1,2-dichloroethane in the gas recycle mode with reduced accumulation of hydrogen, which comprises the following steps:

(A) introduction of ethylene, oxygen and hydrogen chloride as starting materials into an oxychlorination reactor, with the hydrogen chloride comprising hydrogen,
(B) reaction of the starting materials over a catalyst comprising

■ from 3.5 to 12.5% by weight of copper as copper salt
■ from 0.1 to 1.0% by weight of magnesium as magnesium salt
■ from 0.1 to 2.0% by weight of potassium as potassium salt

on a support material
to form 1,2-dichloroethane and water with at least partial conversion of the hydrogen present into water,
(C) work-up of the reactor output and recirculation of the recycle gas to the oxychlorination reactor.

**2.** The process according to claim 1, wherein the catalyst comprises from 7.5 to 9.0% by weight of copper as copper salt, from 0.3 to 0.7% by weight of magnesium as magnesium salt and from 0.6 to 0.9% by weight of potassium as potassium salt on a support material.

**3.** The process according to either claim 1 or 2, wherein the support material of the catalyst is selected from the group consisting of aluminum oxides, alpha-aluminum oxide, beta-aluminum oxide, gamma-aluminum oxide, activated aluminum oxides, silicon oxides such as silicates, silica gels, silicic acid and kieselguhr, graphite, metal oxides, zirconium oxides, zeolites, water glass, pumice, clays, aluminas and mixtures of the abovementioned materials.

**4.** The process according to any of claims 1 to 3, wherein the catalyst comprises gamma-aluminum oxide as support material.

**5.** The process according to any of claims 1 to 4, wherein the catalyst has a BET surface area in the range from 60 to 200 $m^2/g$

**6.** The process according to any of claims 1 to 5, wherein the catalyst has a BET surface area in the range from 80 to 145 $m^2/g$

**7.** The process according to any of claims 1 to 6, wherein the catalyst has a BET surface area in the range from 100 to 130 $m^2/g$.

**8.** The process according to any of claims 1 to 7, wherein the catalyst has a tamped density of greater than 1000 g/l.

**9.** The process according to any of claims 1 to 8, wherein the catalyst has an average particle size ($D_{50}$) in the range from 45 to 75 $\mu$m.

**10.** The process according to any of claims 1 to 9, wherein the starting materials are introduced into the reactor in the molar ratios ethylene from 1.00 to 2.0:hydrogen chloride 2.0:oxygen from 0.5 to 1.0.

**11.** The process according to any of claims 1 to 10, wherein the starting materials are introduced into the reactor in the molar ratios ethylene from 1.55 to 2.0:hydrogen chloride 2.0:oxygen from 0.5 to 0.75.

**12.** The process according to any of claims 1 to 11, wherein the oxychlorination reactor is a fluidized-bed reactor.

**13.** The use of a catalyst comprising

- ■ from 3.5 to 12.5% by weight of copper as copper salt,
- ■ from 0.1 to 1% by weight of magnesium as magnesium salt and
- ■ from 0.1 to 2% by weight of potassium as potassium salt

on a support material
for preventing accumulation of hydrogen in a process for the oxychlorination of ethylene to form 1,2-dichloroethane in the gas recycle mode as described in any of claims 1 to 12.

**Revendications**

**1.** Procédé d'oxychloration d'éthylène en 1,2-dichloroéthane en mode à gaz circulaire avec accumulation réduite d'hydrogène, comprenant les étapes suivantes :

(A) l'introduction d'éthylène, d'oxygène et de chlorure d'hydrogène en tant que réactifs dans un réacteur d'oxychloration, le chlorure d'hydrogène contenant de l'hydrogène,
(B) la mise en réaction des réactifs sur un catalyseur contenant :

- 3,5 à 12,5 % en poids de cuivre sous la forme d'un sel de cuivre,
- 0,1 à 1,0 % en poids de magnésium sous la forme d'un sel de magnésium,
- 0,1 à 2,0 % en poids de potassium sous la forme d'un sel de potassium,

sur une substance support,
pour former du 1,2-dichloroéthane et de l'eau avec transformation au moins partielle de l'hydrogène présent en eau,
(C) le traitement de la sortie du réacteur et le recyclage du gaz circulaire dans le réacteur d'oxychloration.

**2.** Procédé selon la revendication 1, **caractérisé en ce que** le catalyseur contient 7,5 à 9,0 % en poids de cuivre sous la forme d'un sel de cuivre, 0,3 à 0,7 % en poids de magnésium sous la forme d'un sel de magnésium et 0,6 à 0,9 % en poids de potassium sous la forme d'un sel de potassium sur une substance support.

**3.** Procédé selon l'une quelconque des revendications 1 à 2, **caractérisé en ce que** la substance support du catalyseur est choisie dans le groupe constitué par les oxydes d'aluminium, l'oxyde d'aluminium alpha, l'oxyde d'aluminium bêta, l'oxyde d'aluminium gamma, les oxydes d'aluminium activés, les oxydes de silicium tels que les silicates, les gels de silice, la silice et la diatomée, le graphite, les oxydes de métaux, les oxydes de zirconium, les zéolithes, le verre soluble, la pierre ponce, les argiles, les corindons ou les mélanges des substances susmentionnées.

**4.** Procédé selon l'une quelconque des revendications 1 à 3, **caractérisé en ce que** le catalyseur contient de l'oxyde d'aluminium gamma en tant que substance support.

**5.** Procédé selon l'une quelconque des revendications 1 à 4, **caractérisé en ce que** le catalyseur présente une surface BET dans la plage allant de 60 à 200 $m^2$/g.

**6.** Procédé selon l'une quelconque des revendications 1 à 5, **caractérisé en ce que** le catalyseur présente une surface BET dans la plage allant de 80 à 145 $m^2$/g.

**7.** Procédé selon l'une quelconque des revendications 1 à 6, **caractérisé en ce que** le catalyseur présente une surface BET dans la plage allant de 100 à 130 $m^2$/g.

**8.** Procédé selon l'une quelconque des revendications 1 à 7, **caractérisé en ce que** le catalyseur présente une densité tassée supérieure à 1 000 g/l.

**9.** Procédé selon l'une quelconque des revendications 1 à 8, **caractérisé en ce que** le catalyseur présente une taille de particule moyenne (valeur $D_{50}$) dans la plage allant de 45 à 75 $\mu$m.

**10.** Procédé selon l'une quelconque des revendications 1 à 9, **caractérisé en ce que** les réactifs sont introduits dans le réacteur en les rapports molaires éthylène 1,00 à 2,0 : chlorure d'hydrogène 2,0 : oxygène 0,5 à 1,0.

**11.** Procédé selon l'une quelconque des revendications 1 à 10, **caractérisé en ce que** les réactifs sont introduits dans le réacteur en les rapports molaires éthylène 1,55 à 2,0 : chlorure d'hydrogène 2,0 : oxygène 0,5 à 0,75.

**12.** Procédé selon l'une quelconque des revendications 1 à 11, **caractérisé en ce que** le réacteur d'oxychloration est un réacteur à lit fluidisé.

**13.** Utilisation d'un catalyseur contenant :

- 3,5 à 12,5 % en poids de cuivre sous la forme d'un sel de cuivre,
- 0,1 à 1 % en poids de magnésium sous la forme d'un sel de magnésium et
- 0,1 à 2 % en poids de potassium sous la forme d'un sel de potassium,

sur une substance support,
pour empêcher l'accumulation d'hydrogène dans un procédé d'oxychloration d'éthylène en 1,2-dichloroéthane en mode à gaz circulaire tel que décrit dans l'une quelconque des revendications 1 à 12.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 1618701
- EP 1464395 A
- EP 1666145 A